# EUROPEAN PATENT SPECIFICATION

(11) **EP 2 762 052 B1**
(45) Date of publication and mention of the grant of the patent: **26.12.2018**
(21) Application number: 13153747.4
(22) Date of filing: 01.02.2013
(51) Int. Cl.: A47L 15/42, G01N 33/00

(54) **A method of operating a household appliance, and a household appliance, in particular a dishwasher**
Verfahren zum Betreiben eines Haushaltsgerätes und Haushaltsgerät, insbesondere ein Geschirrspüler
Procédé de fonctionnement d'un appareil ménager et appareil ménager, en particulier un lave-vaisselle

(43) Date of publication of application: 06.08.2014
(73) Proprietor: Rheinische Friedrich-Wilhelms-Universität Bonn, 53113 Bonn (DE)
(72) Inventor: Stamminger, Rainer, 53115 Bonn (DE); Boeker, Peter, 53225 Bonn (DE); Gilleßen, Claudia, 50374 Erftstadt (DE)
(74) Representative: Louis Pöhlau Lohrentz

(56) References cited:
- EP-A1- 2 196 576
- WO-A1-2005/047788
- WO-A1-2006/082552
- JP-A- H07 338
- JP-A- 2002 315 716
- JP-A- 2008 307 164
- US-A1- 2010 300 487

## Description

The invention relates to a method of operating a household appliance, in particular a dishwasher, in dependence on the presence of odours due to decomposition of organic material in that household appliance. The invention equally relates to a household appliance according to the preamble of claim 9, which is, e.g. known from JP 2008-307164 A.

In household appliances, deterioration of foodstuffs may occur and lead to arising of various odours. These odours depend on the food residues. Typically, such odours are unpleasant, and it is desired to avoid the occurrence of such odours. For example, if food residues may be present in a dishwasher, e.g. on dishes and cutlery, introduced some time ago, one foresees that the dishwasher becomes automatically active in a manner suitable to reduce odours due to the food residues. Typically in the dishwasher, an additional rinsing step takes place to remove the food residues from the dishes and cutlery.

It is also known to remove the odour itself.

In both cases, there must be a means triggering the action to reduce the odours.

US 2007/0131259 A1 discloses a method for eliminating odours in a dishwasher machine comprising a photocatalytic layer able to convert undesirable odours of organic origin at its surface into harmless substances. To that end, the photocatalytic layer needs to be exposed to UV-light, and air needs to circulate in the washing container. In one embodiment, this exposure of the photocatalytic layer and/or the circulation of the air located in the washing container are either sensor-controlled or time-controlled. In particular, it is disclosed that the sensor designed for this purpose could detect odours.

In JP 2008/307164 A, a dishwasher is disclosed which has two sensors. One sensor detects the smell of left-over food in the dishwasher. A second sensor detects the smell of detergent. The signals of both sensors determine whether or not an additional rinsing and addition of detergent takes place. If much left-over food is determined whilst there is not much detergent, the amount of detergent is increased.

It is extremely difficult to precisely measure all kinds of odours which can arise depending on food residues in household appliances. Usually, for precisely measuring odours, an individual sensor cannot be sufficient.

From the technical field of so-called electronic noses, it is known to use more than three and typically six to ten different sensors each responsive to different gases. If particular conditions are met such as the application of a reference gas, of provision of sample air, if the flux of the gases is controlled, and if humidity is stabilized, all of the sensors provide signals which are subjected to a pattern recognition.

It would be too extremely complicated to use six to ten sensors of an electronic nose and to provide for the conditions to be met in a household appliance such as a dishwasher.

The object of the present invention is thus to provide a method of operating a household appliance in dependence on the presence of odours due to decomposition of organic material in the household appliance as well as to provide a corresponding household appliance, wherein unpleasant odours are reliably detected and/or removed.

The object is solved by a method comprising the features of claim 1 and a household appliance comprising the features of claim 9.

The inventive method of operating a household appliance, in particular a dishwasher, in dependence on the presence of odours due to decomposition of organic material in said household appliance comprises the steps of:
- obtaining first measurement signals by a first sensor responsive (i.e. sensitive) to a first group comprised of at least one first chemical substance which is produced upon decomposition of organic material,
- obtaining second measurement signals by a second sensor responsive (i.e. sensitive) to a second group comprised of at least one second chemical substance which is produced upon decomposition of organic material,
- determining whether or not the first measurement signals fulfill a first pre-determined criterion, and if so,
- determining whether or not the second measurement signals fulfil a second pre-determined criterion, and if so (in case that the second pre-determined criterion is fulfilled), outputting a signal indicating the presence of odours due to decomposition of organic material and/or a signal activating a unit in said household appliance acting to reduce such odours.

The invention is based on the principle to use not only one sensor responsive to chemical substances occurring upon decomposition of organic material, but also a second sensor responsive to at least a chemical substance produced upon the decomposition of organic material.

By this, it is avoided that due to the measurement signals of only one sensor, a cleaning step takes place even if that was not needed; for instance, some sensors (of a first kind) are very sensitive to the environmental conditions such that from the signals itself, one cannot unambiguously gather whether or not gases occur which are very odorous. Other sensors (of a second kind) can measure gases precisely, but only such gases the presence of which in a minimum concentration is not always due to decomposition of organic material. The advantage is in particular provided when in the method, the working principles of how the first and the second measurement signals are obtained by the first and second sensors are different (from each other). These principles might be physical principles, chemical principles or include both aspects ("physico-chemical principles"). Both principles may be physical principles, but different ones, or chemical principles, but different ones, or physico-chemical principles, but different ones, or one principle might be of one kind such as a physical principle and the other of the other kind such as a chemical or physico-chemical principle. If different working principles are used, then causes for giving a "false alarm", i.e. for falsely measuring odours when they are not present, apply to the two different sensors such that it is improbable that such causes will be responsible for both of the sensors outputting measurement signals which would lead to indicating the presence of odours and/or a signal activating a unit acting to reduce such odours.

In a preferred embodiment, the first sensor is a metal oxide semiconductor sensor, which is in particular responsive to volatile organic compounds. Metal oxide semi-conductor sensors are well-known in the field and able to react/to be sensitive to a plurality of different gases. An overview on such sensors is given in the review of George F. Fine, Leon M. Cavanagh, Ayo Afonja and Russel Binions with the title "Metal Oxide Semi-Conductor Gas Sensors in Environmental Monitoring", in: Sensors 2010, 10, pages 5469-5502, ISSN 1424-8220.

The measurement signals output by such sensors (abbreviated as "MOS sensors") always undergo a drift. For that reason, it is an indication of volatile organic compounds (which are produced upon decomposition of organic material) when there is an increase in the measurement signals over a pre-determined time period. For that reason, preferably, the first pre-determined criterion is fulfilled when measurement values provided by the measurement signals increase over a pre-determined period of time in a pre-determined minimum amount (i.e. in at least a pre-determined amount).

It is to be noted that this first criterion, despite of monitoring the time development of the measurement signals, may be fulfilled both a) if at least one of the first chemical substances (the volatile organic compounds) is present in a minimum amount and b) if none of the first chemical substances is present in a respective minimum amount, but if another condition is met (such as, e.g., increase in temperature leading to an increase of the signal height).

In a further preferred embodiment, the second sensor is a non dispersive infrared sensor (NDIR sensor) which is in particular responsive to carbon dioxide.

NDIR sensors are very reliable and are such suited to precisely determine the extent of carbon dioxide (in the household appliance). Since the amount of carbon dioxide increases when there is organic material decomposing, preferably, the second pre-determined criterion is either fulfilled when the measurement value which is provided by the second measurement signals is higher than a pre-determined threshold or is fulfilled when the measurement value which is provided by the second measurement signals is lower than a pre-determined threshold. (This is dependent on the kind of measurement value. Those measurement values which become higher than the pre-determined threshold when the amount of carbon dioxide gas increases may be inverted and such inverted value will become lower than the pre-determined threshold in the same situation.)

It has to be noted that the second criterion is fulfilled if at least one of the second chemical substances is present in a minimum amount (the carbon dioxide), wherein these substances may be present due to decomposition of organic material or due to other reasons.

In combination, it is clear, that the MOS sensors and the NDIR sensor both are not perfectly reliable when detecting the respective chemical substances. This explains the advantage of making use of both sensors and their measurement signals.

In an alternative approach, the second pre-determined criterion is fulfilled when the value of pre-determined function calculated both on the basis of the measurement values provided by the first signals and the measurement values provided by the second signals is higher than a pre-determined threshold value (or when such value of a pre-determined function is lower than a pre-determined threshold value). If for a first pre-determined function, the first condition is met, the second will be met for the inverse function. The function may simply calculate the ratio between the first and second measurement values.

Preferably, in all of the embodiments wherein a threshold value is used in the first and/or second pre-determined criterion, and wherein in these embodiments a signal is output that activates a cleaning unit in the household appliance, it is foreseen that after the completion of a cleaning step, the measurement signals are obtained and stored for the determining of at least one threshold value used when applying at least one of said first and/or second pre-determined criteria.

By this measure, one always obtains to the actual environmental condition the measurement signals in the situation wherein there are no odours (due to the learning step).

The inventive household appliance, in particular a dishwasher, comprises two sensors from which a first sensor is responsive (sensitive) to at least one first chemical substance and a second sensor is responsive to at least one second chemical substance, and this household appliance comprises a cleaning unit acting to remove sources of (unpleasant) odours in the household appliance, and further comprises a control unit receiving measurement signals from the first and second sensors and outputting control signals to said cleaning unit. The household appliance is characterised in that both the first and second sensors are responsive to such chemical substances which occur when organic material is decomposing and that said control unit is adapted to use signals of both the first and second sensors when determining whether or not to output an activating control signal to said cleaning unit.

In other words, the household appliance according to the invention is configured/adapted to perform or make use of the inventive measurement of operating.

In a preferred embodiment, the control unit is adapted to continuously or repeatedly receive measurement signals from the first sensor and to determine whether or not a first pre-determined criterion is fulfilled. In case the pre-determined criterion is fulfilled, the control unit receives measurement signals from the second sensor and determines whether or not a second pre-determined criterion is fulfilled.

Here, the need is avoided to receive by the control unit measurement signals from both of the sensors all of the time, or at least to make use of both of such signals. There is only the need to observe the measurement signals from the first sensor. Only when the first pre-determined criterion is fulfilled, those from the second sensor are observed and made use of.

The further preferred embodiments of the household appliance are indicated in the further dependent claims. The advantages have been explained above.

It is to be noted that the roles of the first and second sensor may be interchanged: In particular, the NDIR sensor might play the role of a first sensor and the metal oxide semi-conductor sensor that of the second sensor. Then of course, the criteria applied are equally interchanged.

The invention is set out in more detail with respect to the drawings, in which
- Fig. 1: shows curves explaining sensor signals in dependence on the time in a situation where no unpleasant odours occur, and
- Fig. 2: shows such signals in case that such unpleasant odours occur, and
- Fig. 3: is a flow chart for explaining a simple embodiment of the inventive method.

In a dishwasher as an example for a household appliance, two kinds of sensors are placed: A first sensor is a metal oxide semi-conductor sensor (MOS sensor) responsive to volatile organic compounds. A second sensor is a NDIR sensor responsive to carbon dioxide.
The household appliance and the sensors are not shown in the figures. The dishwasher may be of any known kind and shape. Usually, the dishwasher comprises a housing (not shown in the figures), in which not only elements to place dishes and cutlery thereon are provided, but wherein equally said two sensors are provided.

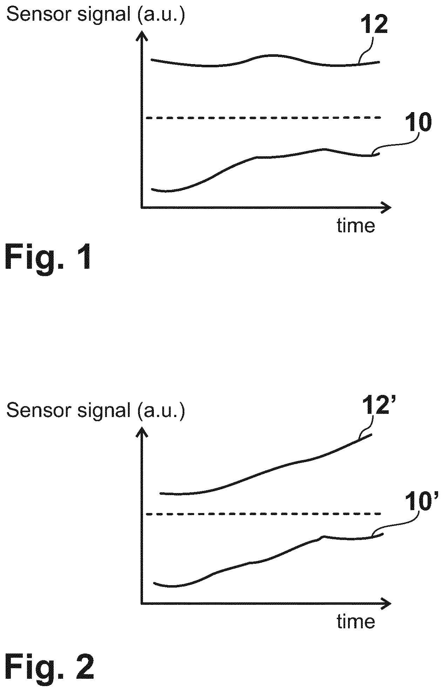

Fig. 1 and 2 show typical curves of the signals output by the sensors (measurement or sensor signal, in arbitrary units).

Fig. 1 shows two curves 10 and 12 under normal conditions without occurrence of unpleasant odours due to a decomposition of organic material. The curve 10 represents the output of the MOS sensor and the curve 12 represents the output of the NDIR sensor. As can be gathered from these figures, the MOS sensor shows a varying sensor signal, the so-called unstable base line. This means that absolute measurement signals are not an indicator of present gas or odour concentrations. The instability of the base line such as curve 10 is a typical and unavoidable characteristic on the measuring principle in a MOS sensor. An increase in the measurement signal of the MOS sensor can be caused not only by increased concentrations of volatile organic compounds, but also by changing external conditions of measurement (such as in particular a temperature or a humidity). These drifts cannot be distinguished from slow changes in the sample gas concentration, as it is typically associated with the slow onset and decay of slow development of volatile organic compounds.

The second sensor, the NDIR sensor, provides information which is independent of volatile organic compounds, since the outputting of measurement signals is based on a completely different physical principle of measurement. Here, a change in the external measurement conditions will not lead to the same signal changes. From fig. 1 one can in particular gather that a drift of the base line of the MOS sensor has to be detected as a mere drift without increase of the amount of volatile organic compounds in case the measurement values provided by the measurement/sensor signal according to curve 12 is remaining constant.

Fig. 2 now shows the situation in which there is indeed a decomposition of organic material: Here, curve 10' shows an increase in the value of measurement signal output by the first sensor (the MOS sensor). At the same time, an increase in the sensor signal output by the second sensor (the NDIR sensor) can be observed, see curve 12'.

Thus, when observing both of these curves at the same time, one can more reliably determine whether or not there are unpleasant odours present.

It is to be noted that an increase in the output signal of the second sensor alone is not reliable enough since the amount of CO₂ may increase due to the presence of people or to combustion processes taking place (when heating a room or in ovens).

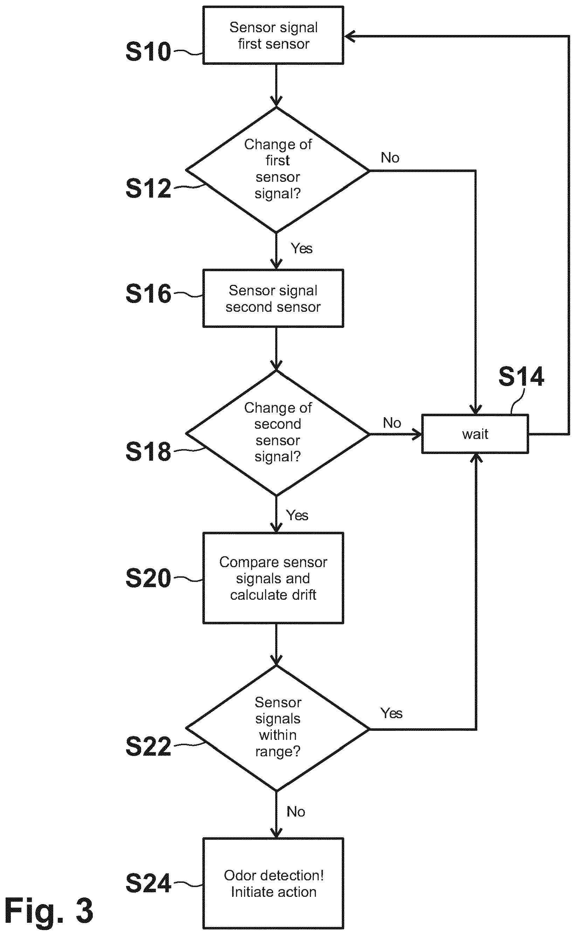

For that reason, a method of the kind as described hereinafter with regard to fig. 3 is used:
In step S10, one receives a sensor signal from a first sensor.
In step S12, a first pre-determined criterion is applied: Does a change in the first sensor signal occur? If this is not the case, the method goes over to step S14, in which it is waited.

Following step S14, the method newly starts with step S10.

In case that in step S12 a change of the first sensor signal is determined according to a pre-determined criterion which for example includes that starting from an initial value, in a pre-determined time period, the signal height has increased by a pre-determined amount, the method goes over to step S16:
In step S16, the sensor signal from the second sensor is received.

Following thereafter in step S18, a second pre-determined criterion is applied: Here, it is determined whether or not the second sensor signal has changed. If this is not the case, once again, step S14 (waiting) is performed prior to returning to step S10.

In case that the second pre-determined criterion is fulfilled which includes for example that a threshold is exceeded, the method continues with step S20: In step S20 the sensor signals are compared, and the drift is in total calculated. Thereafter, in step S22, it is determined whether or not the sensor signals are within a pre-determined range. If so, it is continued with step S14 prior to returning to step S10.

If the sensor signals are not within a pre-determined range, step S24 is the next step: It is now (according to the two pre-determined criteria) clear that an unpleasant odour has been detected, and an action is initiated such as activating a cleaning unit in the dishwasher.

It is to be noted that steps S20 and S22 may be omitted. For example, the determination whether or not the sensor signals are within a range as in step S22 may take place as part of the first and second pre-determined criteria already in complementing steps S12 and S28, respectively.

The roles of the first sensor and second sensor might be interchanged in the flow chart; that means that the order of the steps might be a different one.

## Claims

1. A method of operating a household appliance, in particular a dishwasher, in dependence on the presence of odours due to decomposition of organic material in said household appliance, said method comprising the steps of:
- obtaining (S10) first measurement signals (10, 10') by a first sensor responsive to a first group comprised of at least one first chemical substance which is produced upon decomposition of organic material,
- obtaining (S16) second measurement signals (12, 12') by a second sensor responsive to a second group comprised of at least one second chemical substance which is produced upon decomposition of organic material,
- determining (S12) whether or not the first measurement signals (10, 10') fulfil a first pre-determined criterion, and if so,
- determining (S18) whether or not the second measurement signals (12, 12') fulfil a second pre-determined criterion, and in case that the second pre-determined criterion is fulfilled,
- outputting (S24) a signal indicating the presence of odours due to decomposition of organic material and/or a signal activating a unit in said household appliance acting to reduce such odours.

2. The method according to claim 1,
wherein the working principles of how the first and second measurement signals are obtained by the first and second sensor are different.

3. The method according to claim 1 or claim 2,
wherein the first sensor is a metal oxide semi-conductor sensor, which is in particular responsive to volatile organic compounds.

4. The method according to any of claims 1 or 2,
wherein said first pre-determined criterion is fulfilled when measurement values provided by the first measurement signals (10, 10') increase over a pre-determined period of time in a pre-determined minimum amount.

5. The method according to any of the preceding claims,
wherein said second sensor is a non dispersive infrared sensor, which is in particular responsive to carbon dioxide.

6. The method according to any of the preceding claims,
wherein said second pre-determined criterion is either fulfilled when the measurement values provided by the second measurement signals are higher than a pre-determined threshold or is fulfilled when the measurement values provided by the second measurement signals are lower than a pre-determined threshold.

7. The method according to any one of claims 1 to 5,
wherein said second pre-determined criterion is fulfilled when the value of a pre-determined function calculated both on the basis of the measurement values provided by the first measurement signals (10, 10') and the measurement values provided by the second measurement signals (12, 12') is higher than a pre-determined threshold value or when the value of a pre-determined function calculated both on the basis of the measurement values provided by the first measurement signals and the measurement values provided by the second measurement signals (12, 12') is lower than a pre-determined threshold value.

8. The method of any of claims 3 to 7,
wherein a signal is output that activates a cleaning unit in the household appliance, and wherein after the completion of a cleaning step, the measurement signals are obtained and stored for determining at least one threshold value and when applying at least one of said first and second pre-determined criteria.

9. A household appliance, in particular a dishwasher, comprising two sensors from which a first sensor is responsive to at least one first chemical substance and a second sensor is responsive to at least one second chemical substance and comprising a cleaning unit acting to remove sources of odours in the household appliance, and further comprising a control unit receiving measurement signals (10, 10', 12, 12') for the first and second sensors and outputting control signals to said cleaning unit,
**characterised in that**
both of the first and the second sensors are responsive to such chemical substances which occur when organic material is decomposing and that said control unit is adapted to use signals of both the first and second sensors to perform a method of operating according to any of claims 1-8.

10. The household appliance according to claim 9,
wherein the control unit is adapted to continuously or repeatedly receive measurement signals (10, 10') from the first sensor and to determine whether or not a first pre-determined criterion is fulfilled, wherein in case the first pre-determined criterion is fulfilled, the control unit receives measurement signals (12, 12') from the second sensor and determines whether or not a second pre-determined criterion is fulfilled.

11. Household appliance according to claim 9 or claim 10,
wherein said first sensor is a metal oxide semi-conductor sensor.

12. Household appliance according to claim 11,
wherein said metal oxide semi-conductor sensor is responsive to volatile organic compounds.

13. Household appliance according to any one of claims 9 to 12,
wherein said second sensor is a non dispersive infrared sensor.

14. Household appliance according to claim 13,
wherein said non dispersive infrared sensor is responsive to carbon dioxide.

15. Household appliance according to any of claims 9 to 14, comprising a housing in which the first and second sensors are arranged and/or a sensor housing for each of the first and second sensors.

## Patentansprüche

1. Verfahren zum Betreiben eines Haushaltsgeräts, insbesondere eines Geschirrspülers, in Abhängigkeit vom Vorhandensein von Gerüchen wegen einer Zersetzung organischen Materials in dem Haushaltsgerät, wobei das Verfahren die folgenden Schritte umfasst:
- Erhalten (S10) erster Messsignale (10, 10') durch einen ersten Sensor, der auf eine erste Gruppe anspricht, die aus wenigstens einer ersten chemischen Substanz besteht, die bei einer Zersetzung organischen Materials erzeugt wird,
- Erhalten (S16) zweiter Messsignale (12, 12') durch einen zweiten Sensor, der auf eine zweite Gruppe anspricht, die aus wenigstens einer zweiten chemischen Substanz besteht, die bei einer Zersetzung organischen Materials erzeugt wird,
- Feststellen (S12), ob oder ob nicht die ersten Messsignale (10, 10') ein erstes vorgegebenes Kriterium erfüllen, und falls dies zutrifft,
- Feststellen (S18), ob oder ob nicht die zweiten Messsignale (12, 12') ein zweites vorgegebenes Kriterium erfüllen, und falls das zweite vorgegebene Kriterium erfüllt ist,
- Ausgeben (S24) eines Signals, das das Vorhandensein von Gerüchen wegen einer Zersetzung organischen Materials anzeigt und/oder eines Signals, das eine Einheit in dem Haushaltsgerät aktiviert, die tätig wird, um derartige Gerüche zu verringern.

2. Verfahren nach Anspruch 1,
wobei die Arbeitsprinzipien, auf welche Weise das erste und das zweite Messsignal durch den ersten und den zweiten Sensor erhalten werden, unterschiedlich sind.

3. Verfahren nach Anspruch 1 oder Anspruch 2,
wobei der erste Sensor ein Metalloxid-Halbleitersensor ist, der insbesondere auf flüchtige organische Stoffe anspricht.

4. Verfahren nach einem der Ansprüche 1 oder 2,
wobei das erste vorgegebene Kriterium erfüllt ist, wenn Messwerte, die durch die ersten Messsignale (10, 10') bereitgestellt werden, über eine vorgegebene Zeitspanne um einen vorgegebenen minimalen Betrag ansteigen.

5. Verfahren nach einem der vorhergehenden Ansprüche,
wobei der zweite Sensor ein nicht dispersiver Infrarotsensor ist, der insbesondere auf Kohlenstoffdioxid anspricht.

6. Verfahren nach einem der vorhergehenden Ansprüche,
wobei das zweite vorgegebene Kriterium entweder erfüllt ist, wenn die durch die zweiten Messsignale bereitgestellten Messwerte höher als ein vorgegebener Schwellenwert sind oder erfüllt ist, wenn die durch die zweiten Messsignale bereitgestellten Messwerte niedriger als ein zweiter vorgegebener Schwellenwert sind.

7. Verfahren nach einem der Ansprüche 1 bis 5,
wobei das zweite vorgegebene Kriterium erfüllt ist, wenn der Wert einer vorgegebenen Funktion, der auf der Basis der Messwerte, die durch die ersten Messsignale (10, 10') bereitgestellt werden, und der Messwerte, die durch die zweiten Messsignale (12, 12') bereitgestellt werden, berechnet wird, höher als ein vorgegebener Schwellenwert ist, oder wenn der Wert einer vorgegebenen Funktion, der auf der Basis der Messwerte, die durch die ersten Messsignale (10, 10') bereitgestellt werden und der Messwerte, die durch die zweiten Messsignale (12, 12') bereitgestellt werden, berechnet wird, niedriger als ein vorgegebener Schwellenwert ist.

8. Verfahren nach einem der Ansprüche 3 bis 7,
wobei ein Signal ausgegeben wird, das eine Reinigungseinheit in dem Haushaltsgerät aktiviert, und wobei nach dem Beenden eines Reinigungsschritts die Messsignale erhalten und gespeichert werden, um wenigstens einen Schwellenwert zu bestimmen und zu bestimmen, wann das erste und/oder das zweite vorgegebene Kriterium angewendet wird.

9. Haushaltsgerät, insbesondere ein Geschirrspüler, das zwei Sensoren umfasst, wovon ein erster Sensor auf wenigstens eine erste chemische Substanz anspricht, und ein zweiter Sensor auf wenigstens eine zweite chemische Substanz anspricht, und das eine Reinigungseinheit umfasst, die tätig wird, um Quellen von Gerüchen in dem Haushaltsgerät zu entfernen, und das ferner eine Steuereinheit umfasst, die Messsignale (10, 10', 12, 12') für den ersten und zweiten Sensor empfängt und Steuersignale an die Reinigungseinheit ausgibt,
**dadurch gekennzeichnet, dass**
der erste und der zweite Sensor auf chemische Substanzen ansprechen, die auftreten, wenn sich organisches Material zersetzt, und dass die Steuereinheit ausgelegt ist, Signale des ersten und des zweiten Sensors zu verwenden, um ein Verfahren zum Betreiben nach einem der Ansprüche 1-8 durchzuführen.

10. Haushaltsgerät nach Anspruch 9,
wobei die Steuereinheit ausgelegt ist, ununterbrochen oder wiederholt Messsignale (10, 10') von dem ersten Sensor zu empfangen und festzustellen, ob oder ob nicht ein erstes vorgegebenes Kriterium erfüllt ist, wobei in dem Fall, dass das erste vorgegebene Kriterium erfüllt ist, die Steuereinheit Messsignale (12, 12') von dem zweiten Sensor empfängt und feststellt, ob oder ob nicht ein zweites vorgegebenes Kriterium erfüllt ist.

11. Haushaltsgerät nach Anspruch 9 oder Anspruch 10,
wobei der erste Sensor ein Metalloxid-Halbleitersensor ist.

12. Haushaltsgerät nach Anspruch 11,
wobei der Metalloxid-Halbleitersensor auf flüchtige organische Stoffe anspricht.

13. Haushaltsgerät nach einem der Ansprüche 9 bis 12,
wobei der zweite Sensor ein nicht dispersiver Infrarotsensor ist.

14. Haushaltsgerät nach Anspruch 13,
wobei der nicht dispersive Infrarotsensor auf Kohlenstoffdioxid anspricht.

15. Haushaltsgerät nach einem der Ansprüche 9 bis 14,
das ein Gehäuse, in dem der erste und der zweite Sensor angeordnet sind und/oder ein Sensorgehäuse für den ersten und den zweiten Sensor umfasst.

## Revendications

1. Une méthode de fonctionnement d'un appareil ménager, en particulier d'un lave-vaisselle, en fonction de la présence d'odeurs dues à la décomposition de matière organique dans ledit appareil ménager, ladite méthode comprenant les étapes de :
- obtenir (S10) des premiers signaux de mesure (10, 10') par le biais d'un premier capteur réagissant à un premier groupe constitué d'au moins une première substance chimique qui est produite suite à la décomposition de matière organique ;
- obtenir (S16) de seconds signaux de mesure (12, 12') par le biais d'un second capteur réagissant à un second groupe constitué d'au moins une seconde substance chimique qui est produite suite à la décomposition de matière organique ;
- déterminer (S12) si les premiers signaux de mesure (10, 10') satisfont ou non un premier critère prédéterminé, et le cas échéant :
- déterminer (S18) si les seconds signaux de mesure (12, 12') satisfont ou non un second critère prédéterminé, et dans le cas où le second critère prédéterminé est satisfait,
- fournir en sortie (S24) un signal indiquant la présence d'odeurs dues à la décomposition de matière organique, et/ou un signal activant une unité dans ledit appareil ménager agissant pour réduire de telles odeurs.

2. La méthode selon la revendication 1,
dans laquelle les principes de fonctionnement concernant la manière dont les premier et second signaux de mesure sont obtenus par le premier capteur et le second capteur sont différents.

3. La méthode selon la revendication 1 ou 2,
dans laquelle le premier capteur est un capteur à semi-conducteur à oxyde métallique, lequel réagit en particulier aux composés organiques volatils.

4. La méthode selon l'une quelconque des revendications 1 et 2,
dans laquelle ledit premier critère prédéterminé est satisfait lorsque des valeurs de mesure fournies par les premiers signaux de mesure (10, 10') augmentent sur une période de temps prédéterminée d'une quantité minimale prédéterminée.

5. La méthode selon l'une quelconque des revendications précédentes,
dans laquelle ledit second capteur est un capteur infrarouge non dispersif, lequel réagit en particulier au dioxyde de carbone.

6. La méthode selon l'une quelconque des revendications précédentes,
dans laquelle ledit second critère prédéterminé est soit satisfait lorsque les valeurs de mesure fournies par les seconds signaux de mesure sont supérieures à un seuil prédéterminé, soit satisfait lorsque les valeurs de mesure fournies par les seconds signaux de mesure sont inférieures à un seuil prédéterminé.

7. La méthode selon l'une quelconque des revendications 1 à 5,
dans laquelle ledit second critère prédéterminé est satisfait lorsque la valeur d'une fonction prédéterminée calculée à la fois sur la base des valeurs de mesure fournies par les premiers signaux de mesure (10, 10') et des valeurs de mesure fournies par les seconds signaux de mesure (12, 12') est supérieure à une valeur de seuil prédéterminée, ou lorsque la valeur d'une fonction prédéterminée calculée à la fois sur la base des valeurs de mesure fournies par les premiers signaux de mesure et des valeurs de mesure fournies par les seconds signaux de mesure (12, 12') est inférieure à une valeur de seuil prédéterminée.

8. La méthode selon l'une quelconque des revendications 3 à 7,
dans laquelle un signal est fourni en sortie, lequel active une unité de nettoyage dans l'appareil ménager, et dans lequel, suite à l'achèvement d'une étape de nettoyage, les signaux de mesure sont obtenus et stockés en vue de déterminer au moins une valeur de seuil, et lors de l'application d'au moins l'un desdits premier et second critères prédéterminés.

9. Un appareil ménager, en particulier un lave-vaisselle, comprenant deux capteurs dont un premier capteur réagit à au moins une première substance chimique et un second capteur réagit à au moins une seconde substance chimique, et comprenant une unité de nettoyage agissant pour éliminer des sources d'odeurs dans l'appareil ménager, et comprenant en outre une unité de commande recevant des signaux de mesure (10, 10', 12, 12') pour les premier et second capteurs et fournissant en sortie des signaux de commande à ladite unité de nettoyage ;
**caractérisé en ce que** :
à la fois le premier capteur et le second capteur réagissent à de telles substances chimiques qui sont produites lorsque de la matière organique se décompose, et **en ce que** ladite unité de commande est apte à utiliser des signaux tant du premier capteur que du second capteur pour mettre en oeuvre un procédé de fonctionnement selon l'une quelconque des revendications 1 à 8.

10. L'appareil ménager selon la revendication 9,
dans lequel l'unité de commande est apte à recevoir, en continu ou de manière répétée, des signaux de mesure (10, 10') en provenance du premier capteur, et à déterminer si un premier critère prédéterminé est satisfait ou non, dans lequel, dans le cas où le premier critère prédéterminé est satisfait, l'unité de commande reçoit des signaux de mesure (12, 12') en provenance du second capteur et détermine si un second critère prédéterminé est satisfait ou non.

11. L'appareil ménager selon la revendication 9 ou 10,
dans lequel ledit premier capteur est un capteur à semi-conducteur à oxyde métallique.

12. L'appareil ménager selon la revendication 11,
dans lequel ledit capteur à semi-conducteur à oxyde métallique réagit aux composés organiques volatils.

13. L'appareil ménager selon l'une quelconque des revendications 9 à 12,
dans lequel ledit second capteur est un capteur infrarouge non dispersif.

14. L'appareil ménager selon la revendication 13,
dans lequel ledit capteur infrarouge non dispersif réagit au dioxyde de carbone.

15. L'appareil ménager selon l'une quelconque des revendications 9 à 14, comprenant un boîtier dans lequel sont agencés les premier et second capteurs et/ou un boîtier de capteur pour chacun des premier et second capteurs.
